# EUROPEAN PATENT APPLICATION

(11) **EP 2 742 937 A1**
(43) Date of publication of application: **18.06.2014**
(21) Application number: 12196558.6
(22) Date of filing: 11.12.2012
(51) Int. Cl.: A61K 31/133, A61K 31/366, A61K 45/06, A61P 31/04

(54) **Compositions for use in the treatment of bacterial infections**

(71) Applicant: LIONEX Diagnostics and Therapeutics GmbH, 38126 Braunschweig (DE)
(72) Inventor: Singh,Mahavir, 38124 Braunschweig (DE); Wanas, Hanaa, 38126 Braunschweig (DE); Oehlmann, Wulf, 38126 Braunschweig (DE); Vilaplana, Cristina, 38126 Braunschweig (DE); Cardona, Pere-Joan, 38126 Braunschweig (DE); Palomino, Juan Carlos, 38126 Braunschweig (DE)
(74) Representative: Kröncke, Rolf

(57) **Abstract**

The present invention relates to a pharmaceutical composition comprising a combination of ethambutol with at least one additional anti-bacterial agent, in particular, anti-mycobacterial agent for use in the treatment of bacterial infection, in particular, mycobacterial infection.

## Description

### Introduction:

Prokaryotic DNA-dependent RNA polymerase (RNAP) is a multi-subunit enzyme responsible for transcription in bacteria. It is an attractive target for development of antibacterial agents as it is crucial for bacterial growth and survival, and shows features that make it different from mammalian RNAP. At present, the rifamycins are the only group of RNAP inhibitors (RNAPIs) that have been approved for clinical use (*Chopra*, *2007*).

Rifampicin (RIF) is the most important rifamycin in the treatment of tuberculosis. Other rifamycins of importance are, rifapentine, rifabutin, rifalazil, and rifamycin T9 (*Aristoff et al., 2010).* Some of them have higher anti-tuberculous activity than RIF. The action of rifamycins is not limited against *Mycobacterial tuberculosis*(M. tb) but also against other gram positive bacteria, for example in the treatment of leprosy (*Bujnowski et al.*, *2003*). RIF is a first line anti-TB medication and it is a key component of the initial anti-TB regimen. It is most efficient in inhibiting actively replicating bacteria. It is not useful only in treating active TB, but also in inhibiting latent bacteria; however it is not recommended to be used alone due to high rate of resistance (*Chao & Rubin*, *2010*).

### Mechanism of rifampicin action and resistance:

The mycobacterial RNA polymerase (RNAP) consists of five subunits. The core enzyme is formed of α,α2, β, and β' subunits. The w subunit only binds when the polymerisation is initiated and falls off when the elongation starts. RIF binds to β subunit however, the core can be still assembled to the DNA and the first phosphdiester bonds can be formed. RIF blocks further formation of transcripts after three or four base pairs and RNA elongation is inhibited *(Artsimovitch&Vassylyev, 2006*). More than 96% of RIF-resistant clinical isolates of *M. tb* were found to have mutations in the 81-bp core region of *rpoB* gene encoding the β subunit. (*Musser*, 1995) (*Valim et al., 2000*). These mutations can circumvent RIF binding through steric hinderance or reduced affinity; also the inhibitory signal of the drug can be distorted through allosteric modulation *(Artsimovitch&Vassylyev, 2006).*

Not all mutations within the 81 bp region display the same level of resistance, mutations in codon 526 or codon 531 which represent 65-85% of mutations result in high-level resistance to RIF with MIC >32µg/ml. However, alterations in codons 511, 516, 518, and 522 result in strains that have low-level resistance to RIF and rifapentin but remain susceptible to two other rifamycins, rifabutin and rifalazyn (*Moghazeh et al., 1996*). Rare mutations associated with RIF-resistance have also been found in the amino-terminal region of *rpoB* (*Heep et al., 2000*)

Approximately 90% of RIF-resistant isolates are also resistant to isoniazid, so RIF-resistance could be considered a useful surrogate marker for multidrug resistance and indicating the urgent need to the second line drugs (*Yuen et al.*, *1999*).

### Progress in the development of new RNAP inhibitors:

One of the main requirements in developing any RNAP inhibitor is to have potential novel target sites which are distinct from the RIF-binding site. Any novel drug candidate designed to replace RIF, should possess activity against RIF-resistant mutants.

Many natural products RNAP inhibitors have been reported. These inhibitors include the ripostatins, corallopyronins, myxopyronins, zwittermicins and the pyrrothines, thiolutin and holomycin.

*O'Neill et al., (2000)* concluded that thiolutin, holomycin, corallopyronin A, and ripostatin A likelyto interact with sites of RNAP distinct from rifampicin. They did not observe cross-resistance with genetically defined rifampicin resistance alleles in *Staphylococcus aureus* and these compounds. The absence of pharmacophoric similarity between these compounds and RIF supports also this hypothesis. On the other hand *rpoB* mutants were cross resistant to sorangicin A. Also the results of *Römmele et al., (1990)* and *Xu et al.,* (2005) showed the cross resistance between rifampicin and sorangicin suggesting that sorangicin A binding site is closely related to that of RIF inspite of the difference in their chemical structure.

Using a combination of genetic, biochemical and structural approaches, *(Mukhopadhyay et al. 2008)* showed that myxopyronin interacts with the RNAP "switch region," the hinge that mediates opening and closing of the RNAP active center cleft. They showed that myxopyronin prevents RNAP to interact with promoter DNA. They proposed that myxopyronin inhibits the initiation of transcription by "jamming the hinge" as it prevents opening of the RNAP active-center cleft to permit entry of DNA. The authers provided experimental evidence that the structurally related α-pyrone antibiotic corallopyronin and the structurally unrelated macrocyclic-lactone antibiotic ripostatin inhibit RNAP through the same target and same mechanism. This explained the absence of cross resistance between these substances and rifamycins, as the residues involved in the interaction are not related to the rifamycins-binding site.

With regard to the physiochemical and pharmacokinetic properties, myxopyronin and corallopyronin exhibit insufficient physiochemical properties, and they have high serum protein binding which reduces their active free part in blood. However the RNAP switch region is a very attractive drug target, these substances need to be further developed to improve their physiochemical and pharmacokinetic properties to meet the requirements of an efficient drug (*Haebich&Von Nussbaum*, 2009).

As used herein, the term "comprise" or "comprising", or the term "containing" or "containing" and compares the embodiment of "consist of" or "consisting of".

The invention comprises the following embodiments:
1. Pharmaceutical composition comprising a combination of ethambutol with at least one additional anti-bacterial agent, in particular, anti-mycobacterial agent for use in the treatment of bacterial infection, in particular, mycobacterial infection.
2. The pharmaceutical composition according to embodiment 1 for use in the treatment of bacterial infection, in particular, mycobacterial infection with a multi drug resistant strain.
3. The pharmaceutical composition according to embodiment 1 or 2 for use in the treatment of bacterial infection, in particular, mycobacterial infection of an individual infected with a bacterial strain, in particular, mycobacterial strain, being resistant against rifampicin treatment.
4. The pharmaceutical composition according to any one of embodiments 1 to 3 for use in the treatment of infection with ethambutol sensitive bacterial, in particular, mycobacterial, strains.
5. The pharmaceutical composition according to any one of the preceding embodiments, wherein the at least one additional anti-bacterial agent, in particular, anti-mycobacterial agent is a 2-pyrone containing antibiotic.
6. The pharmaceutical composition according to embodiment 5 for use in the treatment of bacterial infection, in particular, mycobacterial infection wherein the at least one additional anti-bacterial agent is a compound according to formula I 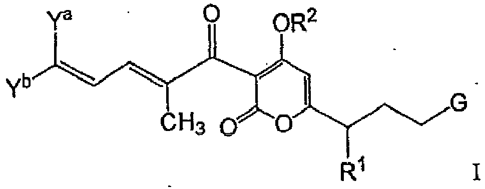 or a pharmaceutically acceptable salt thereof, wherein:
   R1 is hydrogen or-CH3;
   R2 is hydrogen or-C1-C6 straight or branched alkyl;
   one of Ya and Yb is hydrogen or C1-C4 straight alkyl, and the other of Ya and Yb is C1-C10 straight or branched alkyl, C2-C12 straight or branched hydroxyalkyl, C2-C12 straight or branched alkenyl, C2-C12 straight or branched hydroxyalkenyl, phenyl, C7-C12 (aryl)hydroxyalkyl, C6-C12 heteroalkyl, C6-C12
   (heteroaryl)hydroxyalkyl, or Ya and Yb are taken together with their intervening atom to form a 4-6 membered ring having 0-1 ring heteroatoms selected from nitrogen, oxygen or sulphur, said ring optionally substituted by one or two C1-C6 alkyl, C2-C6 alkenyl or hydroxyalkyl groups, wherein an alkyl, aryl or heteroaryl moiety of Ya and Yb optionally is substituted with 1-3 groups independently selected from halo, -C1-C6 hydroxyalkyl, -C1-C4 alkoxy, -C1-C4 trifluororalkoxy, - CN, -C1-C4 alkoxycarbonyl, -C1-C4 alkylcarbonyl, -S(C1-C4 alkyl), and -SO2(C1-C4 alkyl);
   G is -CH=CH-NHC(O)-R3, -CH=CH-NHC(S)-R3, -CH2CH2NHC(O)-R3, -CH2CH2NHC(S)-R3, -CH2NHNHC(O)-R3, or -CH2NHNHC(S)-R3;
   each R3 is independently C1-C6 alkyl, -O(C1-C6 alkyl), -S(C1-C6 alkyl), or-N(R4)2;
      and
   each R4 is independently hydrogen or -C1-C6 alkyl;
7. The pharmceutical composition according to any one of embodiments 1 to 6 wherein the at least one additional anti-bacterial, in particular, anti-mycobacterial agent is an agent selected from ripustatine..(bitte erganzen).
8. The pharmaceutical composition according to any one of the preceding embodiments for use in the treatment of mycobacterial infection wherein the composition comprises a combination of myxopyronine A or B with ethambutol.
9. A method of improving anti-bacterial activity, in particular, bactericidal or bacteriostatic activity of a drug by combining said drug with an effective concentration of ethambutol.
10. The method according to embodiment 9 for improving mycobacterial activity against mycobacteria of an anti-mycobacterial drug by combining it with an effective concentration of ethambutol.
11. A method of treating an individual afflicted with a bacterial infection, in particular, a mycobacterial infection including the step of administering a pharmaceutical composition according to any one of claims 1 to 8 to said individual.
12. The method according to embodiment 11 or the pharmaceutical composition for use in the treatment of bacterial infection, in particular, mycobacterial infection according to any one of embodiments 1 to 8 wherein the active agents are adapted to be administered simultaneously, separately or sequentially.

In preferred embodiments, the pharmaceutical composition further comprises pharmaceutical additives or auxiliary substances, preferably, a pharmaceutically acceptable carrier, excipient or diluent.

The term "pharmaceutical composition" means a composition suitable for pharmaceutical use in a subject or individual, including an animal or human. A pharmaceutical composition generally comprises an effective amount of an active agent or ingredient and a carrier, including e.g. a pharmaceutically acceptable carrier.

A "therapeutic treatment" is a treatment administered to a subject or an individual to display symptoms or signs of pathology, disease, or disorder in which treatment is administered into the subject for the purpose of diminishing or eliminating those signs or symptoms of pathology, disease or disorder.

As used herein, the term "carrier" refers to a diluent, adjuvant, excipient or vehicle.

The composition comprising the compounds according to the present invention will be formulated, dosed, and administered in a fashion consistent with good medical practice. Factors for consideration in this context include the stage of the particular disease or disorder being treated, the particular mammal being treated, the clinical condition of the individual subject, the site of delivery of the agent, the method of administration, the scheduling of administration, and other factors known to medical practitioners. The effective amount of the agent to be administered will be governed by such considerations. The key factor in selecting an appropriate dose and scheduling is the result obtained, as indicated above. The compounds according to the present invention are administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated.

"Therapeutically- or pharmaceutically-effective amount" as applied to the compositions of the instant invention refers to the amount of composition sufficient to induce a desired biological result. That result can be alleviation of the signs and markers, symptoms, or causes of a disease, or any other desired alteration of a biological system.

In vitro assays as well as animal models may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition comprising the active ingredient according to the present invention in form of salts and solvates thereof to an individual.

The pharmaceutical composition according to the present invention comprises the active ingredient as described herein and, optionally, a pharmaceutical acceptable carrier. Such pharmaceutical compositions comprise a therapeutically effective amount of the active ingredient as defined herein and, optionally, a pharmaceutically acceptable carrier. The pharmaceutical composition may be administered with a physiologically acceptable carrier to a patient, as described herein. Acceptable means that the carrier be acceptable in the sense of being compatible with the other ingredients of the composition and not be deleterious to the recipient thereof. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, aerosols and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds, salts or solvates thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

It is preferred that the pharmaceutical composition is for use in treating mycobacterial infection, in particular, infection with *Mycobacterium tuberculosis*, *Mycobacterium bovis*, *Mycobacterium lepare, Mycobacterium ulcerans.*

### Brief description of the drawings

**Fig. (1****): Schematic presentation of the RNAP assay**. A, B, C and D represent the sequential steps of the assay. NTP: Nucleotides (ATP, UTP, GTP&CTP), PPi: Pyrophosphate, APS: adenosine 5' phosphosulfate, Luc: Luciferin. (Bhuju, 2009).
**Fig. (2****):Screening of different substances in RNAP assay**: RNAP assay was used to predict the inhibitory effect of different substances at different concentrations along with positive control that contained all constituents of the reaction, negative control in which RNAP was excluded and rifampicin (RIF) as a control of RNAP inhibition. The Y-axis represents the RLU and at the X-axis the substances are shown.

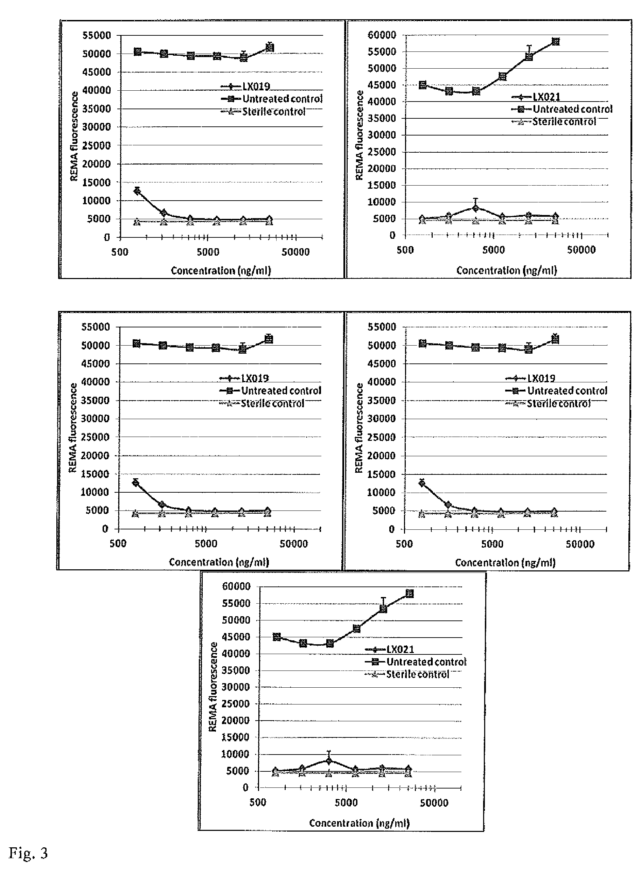

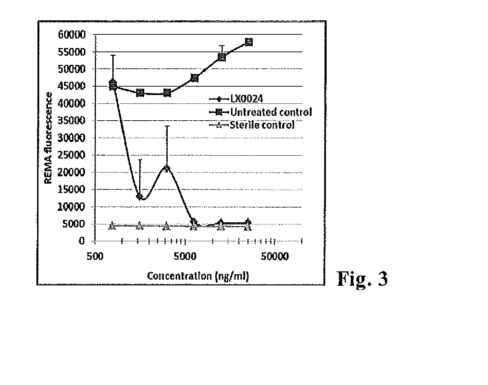

REMA was used to determine the MICs of different combinations of myxopyronin and ethambutol against *Mycobacterium bovis* BCG (Fig 3).

**Fig (3****): Resazurin microplate assay (REMA) with RNAP inhibitors:** Serial 2-fold dilutions of each cocktail were prepared in a 96-well plate, and their minimal inhibitory concentrations (MICs) were evaluated in REMA. The experiment includeded untreated controls treated only with the vehicle and sterile controls contain no bacteria. Results show the correlations between drug concentration and fluorescence.

The invention is further illustrated by the following examples.

### RNAP assay as a tool:

RNAP assay can be used as a screening tool to predict the inhibitory effect of different substances on RNAP. Historically, there are some assays that used to quantify the activity of RNAP. The one utilizing radioisotopically tagged ribonucleotides, as prescribed by *Daniel et al., (1975), McClure(1980), Wu et al.,* (*1997*), has the disadvantages of high reagent cost, high disposal cost of isotopically tagged materials, short shelf life of isotopes and strict monitoring of the laboratory area where isotopes are used. On the other hand, chemically modified nucleotides like fluorescent derivative of nucleotide (*Bhat et al.*, *2006*) or other derivative of nucleotide (*Vassiliou et al.*, *2000*) are not as suitable substrates as the natural nucleotides. *Kuhlman et al.*, (*2004*) used the florescent RiboGreen dye to detect RNA as an end product of transcription. RiboGreen is enhanced by both DNA and RNA, so Dnase digestion and ultrafiltration steps should be added to remove the DNA template. We developed a non radioactive assay in which the normal nucleotides were utilized. The assay is based on measuring the pyrophosphate (PPi) that is released as an end product of transcription. It depends on converting PPi using ATP sulfurylase into ATP that could be measured by luciferin-Luciferase system (*Bhuju*, *2009*).

In this embodiment we present evidence and example of a novel tuberculosis drug cocktail which efficiently inhibits the growth of *Mycobacterium tuberculosis*. We further show how Myxopyronin, which alone is not an efficient drug, can be made effective for killing the tuberculosis pathogen.

### Materials & method:

### RNAP assay:

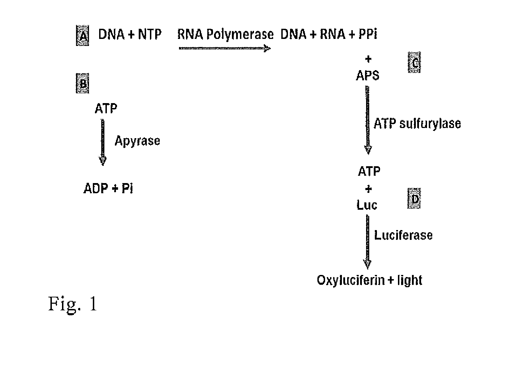

The assay was done as described by *Bhuju*, (*2009*) with few modifications.The assay depends on measuring the activity of RNA polymerase (RNAP) through quantification of the amount of released pyrophosphate (PPi) as an end product of transcription. PPi is transformed by ATP sylfurylase into ATP that could be measured using the Luciferin-Luciferase system. To lower the background of the reaction, apyrase is used to degrade the remaining nucleotides after the RNAP reaction before converting PPi into ATP (Fig. 1).

### ➢ Chemicals used for the assay:

**Table (1): Chemicals used for RNAP assay:**

| **Substance** | **Company** |
|---|---|
| Adenosine 5'-phosphosulfate sodium salt | (Sigma Aldrich) |
| Apyrase | (NEB) |
| ATP Determination Kit | (Biaffin GmbH) |
| ATP Sulfurylase | (NEB) |
| CTP, 100mM Solution | (Fermentas) |
| GTP, 100mM Solution | (Fermentas) |
| UTP, 100mM Solution | (Fermentas) |
| ATP, 100mM Solution | (Fermentas) |
| Manganese Chloride tetrahydrate | (Sigma Aldrich) |
| *E. coli* RNAP core enzyme | (Biozym) |
| Sodium Pyrophosphate Decahydrate | (Sigma Aldrich) |
| Rifampicin | (Sigma Aldrich) |
| Test compounds | (Lionex) |

### ➢ DNA preparation for the assay:

pUC18 was purified using Maxi prep according to manufacturer's instruction. A colony of *E. coli* Top 10F' with pUC18 was cultivated in 100 ml LB medium supplemented with 100 ig/ml ampicillin. After centrifugation at 6000 x g for 15 min at 4°C,the pellet was homogenously resuspended in 10 ml Buffer P1 (Resuspension buffer). Then 10 ml Buffer P2 (Lysis buffer) was added and mixed by vigorously inverting 4-6 times. The mixture was left to be incubated at room temperature for 5 min to lyse the cells. QIAfilter cartridge was prepared by screwing the cap onto the outlet nozzle of the QIAfilter Maxi cartridge. After the incubation period, 19 ml of chilled Buffer P3 (Neutralization buffer) was added and mixed by vigorously inverting 4-6 times in order to neutralize the effect of lysis. And to clear the bacterial lysate, it was poured into the barrel of the QIAfliter cartridge and left at room temperature for 10 min. The cap from the QIAfilter cartridge outlet nozzle was removed and carefully inserted into the QIAfilter Maxi cartridge and the cell lysate was filtered. Buffer ER (2.5 ml) was added to the filtrate. Mixing was carried out by inverting the tube for 10 times and the mixture was incubated on ice for 30 min. equilibration of Qiagen-tip 500 was done by adding 10 ml of Buffer QBT and leaving the column to empty by gravity flow. The filtered lysate was applied to the QIAGEN-tip to allow the plasmid to bind to the resin. Unnecessary contaminants were removed by washing the QIAGEN-tip with 30 ml of Buffer QC (Wash buffer). Elution of bound DNA was done with 15 ml of Buffer QF (Elution buffer). The eluted DNA was precipitated by adding 10.5 ml of isopropanol. After centrifugation at 15,000 x g for 30 min at 4°C, the supernatant was decanted. The DNA pellet was washed with 5 ml of 70%ethanol and the supernatant was decanted after centrifugation at 15,000 x g for 10 min. The pellet was air-dried and the DNA was redissolved in 500 µl distilled water.

The size of the plasmid was determined by analysis on 1% or agarose gel. Agarose was dissolved in 1x TAE buffer. DNA was loaded with 6-fold loading buffer (Fermentas) onto the gel using 1 x TAE as running buffer. The electrophoresis was performed at 80 - 100 Volts for 30-45 minutes. The agarose gel was stained in a GelStar Nucleic Acid Stain (Lonza Verviers) in 1x TAE for 10 min. DNA was visualized using Dark Reader transilluminator (Clare Research) and Photography of the agarose gel was done under UV light with Fluor-S Multilmager (Bio-Rad). The DNA concentration was measured using Qubit quantification fluorometer and Quant-iT reagents (Invitrogen) and finally, the concentration was adjusted to 250µg/ml.

**Table (2): Composition of 50X TAE buffer.**

| | |
|---|---|
| Tris-Base | 242 g |
| Glacial acetic acid | 57.1 ml |
| 0.5 M EDTA (pH 8.0) | 100 ml |
| Water up to | 1000 ml |

### ➢ Procedure of the assay:

1) ATP alone was measured with the Luciferin-Luciferase system as the first standard method (using ATP 1 nm, 10nm, 100nm, 1µM, 10µM solutions).
2) The ability of ATP Sulfurylase to utilize PPi and convert it into ATP was confirmed by using PPi as a substrate: The positive reaction contained 10 ìM of PPi, 5 iM adenosine-5' phosphosulfate, 30 mU ATP Sulfurylase. The positive reaction was repeated with the presence of RIF 10 µg/ml and in the negative reaction, PPi was excluded. The reaction was run in a thermocycler for 10 min at 30°C. Then denaturation of the enzyme was performed at 85°C for 10 min. lastly net ATP produced during the whole reaction was measured using ATP kit.
3) The whole assay was performed as follows (all incubation and denaturating steps were carried in a thermocycler :
   - The RNAP reaction mixture contained 2U of *E. coli* RNAP core enzyme, 500 ng pUC18 plasmid DNA in 10 mM MgCl2, 1.5 mM MnCl2, 0.1 mM EDTA, 50 mMNaCl, 20 mMHepes, pH 8.0 and 25 µM of each NTPs (ATP, UTP, GTP and CTP). The DNA was excluded in the negative reaction and the positive control is repeated with the presence of RIF 10 µg/ml. The mixture then incubated for 30 minutes at 37°C for RNAP to synthesize RNA. This results in at least 90% maximal RNA synthesis (*Kuhlman et al. 2004*).
   - 50 mU of apyrase was added to degrade the residual NTPs present in the reaction mixture. The reaction was carried out for 15 min at 30°C. Then denaturating of the enzyme was done for 10 min at 85°C.
   - (APS) adenosine-5'-phosphosulfate (15 µM) and ATP Sulfurylase (30 mU) were added to transform PPi into ATP. This step was carried out for 15 min at 30°C.
   - A final denaturating of the enzymes present in the whole reaction was performed at 85°C for 10 min.
   - Finally, the amount of ATP produced during the whole reaction was evaluated with ATP kit (Biaffin GmbH). ATP catalyzes the conversion of luciferin into oxyluciferin with production of visible light.
   - Luminance was measured with POLARstar OPTIMA (BMG Labtech) in half area white plates (Costar). The relative light unit detected from a reaction mixture corresponds to the amount of ATP present in the reaction mixture, which reflects the amount of PPi produced during the polymerization reaction of RNAP.
4) The whole assay was repeated with the presence of different concentrations of the test compounds.

### Minimal inhibitory concentration (MIC):

The minimal inhibitory concentrations (MICs) of the substances were determined using resazurin microtiter assay (REMA) as described by Palomino (*Palomino et al.,* 2002) in 96 well microplate. Resazurin is a blue dye, itself nonfluorescent until it is reduced to the pink colored and highly red fluorescent resorufin by living cells. Resazurin sodium salt powders (Sigma) were prepared at 0.01% (wt/vol) in distilled water and filter sterilized. It was stored at 4°C and used within 1 week. The assay was performed in 7H9 medium (BD) supplemented with 10% ADC enrichment medium (Fluka), 0.2% glycerol (Roth), 0.02% Tween-80 (Sigma).

Two-fold serial dilutions of the tested compounds were prepared in 96 well plates in 7H9 medium with volume 100µl. When the bacteria reached early exponential phase (OD₅₅₀ =0.3), it was sonicated in ice bath for 30 sec and then diluted 1:20, and 100 µl was used as an inoculum. Growth controls containing no antibiotic and sterility controls without inoculation were also included. The plates were covered, sealed, and incubated at 37°C in the normal atmosphere. After 7 days, 30 µl of resazurin solution was added to each well and the plates were further incubated overnight at 37°C. The change of color was assessed; a change from blue to pink indicated reduction of resazurin and thus bacterial growth. The MIC is defined as the lowest substance concentration that prevents this color change. Dose-response curves were drawn by measuring the florescence in black plates with excitation at 560nm and emission at 590nm.

To determine the effect of protein binding on the *in vitro* MIC, the test was performed in medium that contains 50% fetal bovine serum (FBS).

### Assessment of in vitro cellular toxicity profile:

### ➢ Cell lines and media:

Cell lines were kindly provided by Dr. Florence Sasse, Helmholtz Center for Infection Research, Braunschweig, Germany. L-929 was from Lionex GmbH, Braunschweig, Germany. Cells were grown at 37°C and 10% CO₂ in the following media:
- L-929 cells (mouse connective tissue fibroblast)Dulbecco's modified Eagle's medium (DMEM)
- PtK2 cells (rat kangaroo kidney epithelial cells): Minimum Essential Medium (MEM) medium, supplemented with 1 % non-essential amino acids and 1 % glutamax, Invitrogen.

All media were supplemented with 10 % fetal calf serum (Gibco).

**Table (3): Apparatus & materials used for cell culture**

| **Item** | **Details** |
|---|---|
| Incubator | CO₂-Auto-Zero, Thermo Scientific |
| Microscope | Axiovert 35, Zeiss, Germany |
| Hemocytometer | Neubauer improved, Assistent Germany |
| Multi-platereader | Wallac, 1420 Victor Multilabel Counter |
| Vortex | Heidolph, REAX 1 R, Germany |
| Digital multichannel | Matrix Technologies Corp, Thermo Scientific |
| Pipette | |
| FacScan | BD LSR II |
| Flourescent Microscope | Zeiss, Germany |
| 96 wellplates | Falcon, Becton Dickinson, USA |
| Scraper | Nalgenunc international, nunc, USA |
| Resevoir | Carl Roth, Germany |
| 4-well plates | Nalgenunc international, nunc, USA |
| Round coverslips | Thermoscientific |
| Trypsin | Invitrogen |
| 0.25 % trypsin/EDTA | Invitrogen |
| Earle's balanced salt | Gibco |
| solution | |

### In vitro toxicity testing:

The effects of the tested compounds on cell proliferation and the acute toxic effects were assayed *in vitro* on different mammalian cell lines.The metabolic activity was measured by means of (MTT) cell survival colorimetric assay. MTT (3-[4,5-dimethylthiazol-2-yl]-2,5-diphenyl tetrazolium bromide) is a yellow dye that was used to measure the metabolic activity of cells which are capable of reducing it by dehydrogenases to a violet formazan product.

### ➢ Solutions:

### - Phosphate buffered saline (PBS):

PBS solution (pH = 7.45) was prepared by dissolving one PBS tablet (Gibco) in 500 ml distilled water.

### - Acidified isopropanol:

0.4 ml of concentrated HCl was added to 100 ml isopropanol.

### - (MTT) dye:

1 g of MTT (98%, Sigma Aldrich) powder was dissolved in 200 ml of PBS and used directly without dilution. This solution was stored below 4°C, in the dark and was used within two weeks.

### ➢ Steps:

### A) Effect on proliferation:

60 µl of serial dilutions of the test compounds were added to 120 µl aliquots of a cell suspension (50,000 cells/ml) in 96-well microplates then incubated 5 days at 37°C and 10% CO₂. 20 µl MTT dye were added to a final concentration of 0.5 mg/ml. After 2 h the precipitate of formazan crystals was centrifuged, and the supernatant discarded. The precipitate was washed two times with 100 µl PBS and dissolved in 100 µl of the acidified propanol. The plates were stirred for 20 minutes on a microtitre plate shaker. The resulting colour was measured at 590 nm using a multi-plate reader.

### B) Acute toxic effect:

When a substance showed an anti-proliferative effect, the acute toxic effect was evaluated as follows. 120 µl aliquots of a cell suspension (100,000-200,000 cells/ml) in 96-well microplates were incubated at 37°C and 10% CO₂ and allowed to grow for two days to stop the cell propagation. Then 60 µl of serial dilutions of the test compounds were added and incubated for further 24h. After 24h incubation at 37°C and 10% CO₂ the MTT assay was performed as described before.

All investigations were carried out in two parallel experiments. Results of cytotoxicity assays were expressed as the percentage of cellular viability. The IC₅₀ values were determined as the concentrations of tested compounds, at which cell samples developed 50% of the absorbance of untreated control cells as estimated from the dose-response curves,

### Immunocytochemistry (ICC):

The effects of substances on the phenotypical changes of cell shape and adhesion were investigated by the technique of immunocytochemistry (ICC).ICC is a technique used to assess the presence of a specific protein or antigen in cultured cells by using a specific primary antibody, which binds to this protein. And by using a florescent labeled secondary antibody, cells can be visualized under microscope. By this method we can visualize different structures in the cell as the endoplasmic reticulum (ER), microtubules and actin stress fibers. And by staining normal controls of untreated cells we can predict the morphological changes that could be produced by treatment with different substances. Actin filaments, microtubules and the ER were investigated upon treatment of the potoroo cells (PtK2) cells with/without test compounds.

### Reagents:

- Ice cold MeOH/acetone (1:1)
- Paraformaldehyde, Sigma aldrich: 3.7 % in PBS
- PBS
- Triton X-100, Sigma aldrich: 0.1 % in PBS
- Anti-fade solution with DAPI, (Invitrogen)
- Primary and secondary antibodies

### Procedure:

PtK2 cells were grown on cover slips in 4-well plates semi-confluent cells were treated with test compounds and the plates were incubated over the night. According to the type of the antibody that will be used, the cells were fixed either with cold methanol/acetone for 10 min or with 3.7% paraformaldehyde for 10 min followed with Triton X-100 (0.1%) for 5 min. Then the cells were washed with PBS and the primary antibody was added and incubated for 45 min and washed with PBS. Secondary antibody was then added to the cells and incubated for additional 45 min. After washing with PBS, Cover slips were mounted in anti-fade mounting medium with DAPI. Images were taken with a CCD camera attached to a flourescence microscope. The following antibodies were used:
- endoplasmic reticulum (*after MeOH*/*acetone fixation*)
   o Primary antibody: anti GRP-94, thermoscientific
   o Secondary antibody : Alexaflour goat anti-rat 488, Invitrogen
- tubulin (*after MeOH*/*acetone fixation*)
   o Primary antibody: α-tubulin, (Sigma)
   o Secondary antibody: Alexaflour 488 anti-mouse, (Invitrogen)
- *Actin; (after formalin-triton X- 100 fixation)*
   o Phalloidin Ph594, (Molecular probe)

### Minimal inhibitory concentration of different combinations:

| | | |
|---|---|---|
| Cocktail designation | Substance | Substance |

Different cominations of Myxopyronin A and Ethambutol were made and are shown below:
Substance E= Ethambutol
Substance M= Myxopyronin A
Substance C= Corallopyronin

Stock solutions of the cocktails. These were diluted in serial dilutions and tested.

| | | |
|---|---|---|
| Lx001 | E 1 mg/mL | C 1 mg/mL |
| Lx002 | E 1 mg/mL | C 1 mg/mL |
| Lx003 | E 1 mg/mL | M 1 mg/mL |
| **Lx004** | **E 1 mg/mL** | **M 1 mg/mL** |
| Lx005 | E 1 mg/mL | RS 1 mg/mL |
| Lx018 | E 500µg/ml | M 500 µg/ml |
| Lx019 | E 250 µg/ml | M 500 µg/ml |
| Lx021 | E500 µg/ml | M 50 µg/ml |
| Lx024 | E500 µg/ml | M 5µg/ml |

The results with cocktails in bold in the table above are shown below as examples:

### Results:

The RNAP assay was validated using different ATP concentrations and by positive and negative controls.

### Screening potential drug candidates using the RNAP assay:

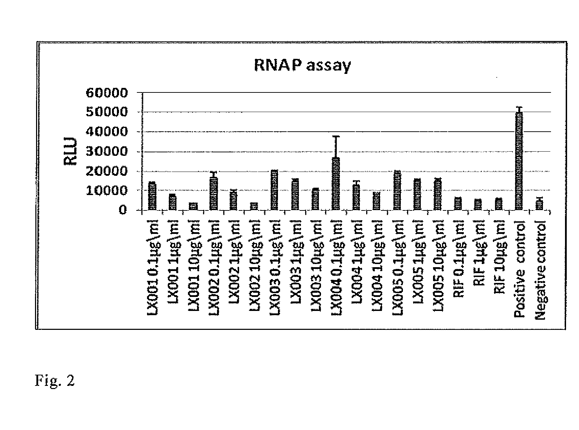

The inhibitory effect of different substances and different drug cocktails called LX cocktails was evaluated in the RNAP assay (Fig. 2). Almost all cocktails inhibited the RNAP in a dose related manner.

### Die LX Cocktaile müssen benannt werden! Sonst keine Offenbarungl

### Evaluating the in vitro inhibitory profile and cellular toxicity of RNAP inhibitors (RNAPIs).

### Synergistic effect between RNAP inhibitors and ethambutol:

The MICs for the individual RNAPI, corallopyronin, ripostatin and myxopyronin A against *M. tuberculosis* H37Rv were evaluated in REMA as described above. Ethambutol is a commercially available anti-TB drug which functions through inhibiting the cell wall. Our novel idea was develop drug cocktails using a given new drug and ethambutol followed by testing their synergistic effects in different test systems. For example, a cellular toxicity assay using cell lines using 7H9 medium containing sub-MIC of ethambutol to calculate the fractional inhibitory concentration (FIC). The synergism was considered when FIC is less than 1. The SI was calculated by dividing the IC₅₀ obtained from the MTT assay over the *in vitro* MIC of each individual substance as shown in the following table.

**Table (4) Synergistic effect between the RNAP inhibitors and ethambutol:**

| | **MIC (µg/ml) against *M. tuberculosis* H37Rv** | | ***In vitro* toxicity with L929 cell line** | |
|---|---|---|---|---|
| | **Individual substance** | **With medium containing ethambutol^{b}** | **IC₅₀ (µg/ml)^{a}** | **SI** |
| **Myxopyronin A** | 6.25 | 3.125 | >100 | >16 |
| **Corallopyronin** | 12.5 | 12.5 | 25 | 2 |
| **Ripostatin** | no inhibition up to 25 µg/ml | no inhibition up to 25 µg/ml | >100 | |
| **Ethambutol** | 1.6 | | >100 | >62. 5 |

| | | | | |
|---|---|---|---|---|
| *^{a}* MTT assay after 5 days. *^{b}* Ethambutol at Conc. 0.3 µg/ml | | | | |

Myxopyronin did not inhibit L929 cell proliferation at all tested concentrations with SI more than 16. On the other hand corallopyronin exhibited low SI and ripostatin did not inhibit the growth of *M. tuberculosis* H37Rv up to concentration of 25 µg/ml. The results showed surprisingly strong the synergistic effect between myxopyronin and ethambutol. The calculated FIC was less than 1.

### FIC = MIC ethambutol in combination/MIC ethambutol alone + MIC myxopyronin in combination/MIC myxopyronin alone = 0.3/1.6 + 3.125/6.25 = 0.64 (FIC less than 1 means synergism)

### Effect on cell viability and selectivity index:

As the different combinations inhibited the mycobacterium at low concentrations, they were further tested in MTT assay with L929 cells to determine the effect on cell viability and to calculate the selectivity index as shown in (table-5).

**Table (5) Minimal inhibitory concentration (MIC), IC₅₀ and selectivity index (SI) of different cocktails:**

| | **MIC** **(µg/ml)^{a}** | **IC₅₀** **(µg/ml)after 5 days^{b}** | **SI** **(^{c}IC₅₀/MIC)** |
|---|---|---|---|
| **LX004** | 1.5 | 35 | 23.3 |
| **LX018** | 1.5 | >100 | > 66.7 |
| **LX019** | 1.5 | >100 | > 66.7 |
| **LX021** | <0.8 | >100 | >125 |
| **LX024** | 6.5 | >100 | > 15.4 |

| | | | |
|---|---|---|---|
| ^{a} MIC was calculated as the dose that inhibited more than 90 % of *M. bovis*BCG growth in REMA. ^{b} in MTT assy with L929 cell line.^{c} IC₅₀ after 5 days. | | | |

The IC₅₀ and MIC data, as described before, were used to calculate the SI as an estimate of the therapeutic window. All combinations did not inhibit cell proliferation at all tested concentrations except LX004. The combinations between Myxopyronin A and Ethambutol inhibited the bacterial growth at lower concentrations at the same time they showed high SI, even LX004 it has also wide safety margin.

### Effect on cell morphology:

Phenotypical changes in the nucleus, endoplasmic reticulum, microtubules (Fig. 10) and actin stress fibres (see appendix) were monitored in potoroo cells (PtK2) treated with 20 µg/ml of the individual substances.

Myxopyronin and ethambutol had no effect on the cell morphology. It was noticed that corallopyronin upon long storage produced fragmentation of the nucleus. And as revealed from the staining of the microtubules, it induced abnormal mitotic spindles.

Furthermore the diferent combinations between myxopyronin and ethambutol were also evaluated with regard their effect on cell morphology. None of the cocktails containing Myxopyronin A and Ethambutol ombinations did not produce any abnormality of the cellular structures, nuclus, endoplasmic reticulum, microtubules and stress fibres.

### Effect of protein binding on in vitro MIC:

Binding to proteins in plasma is a very important factor that can influence the *in vivo* antimicrobial activity. For this reason, the MICs of Myxopyronin, ethambutol and their combination in LX004 and LX018 against *M. bovis* BCG were investigated using resazurin assay in 100 % medium and with the presence of 50 % fetal bovine serum (FBS).

**Table (6): Effect of protein binding on in vitro MIC.**

| | **MIC in 100% medium** | **With medium containing 50 % FBS** |
|---|---|---|
| **Myxopyronin** | 6.25 | no inhibition up to 25 µg/ml |
| **Ethambutol** | 2.5 | 2.5 |
| **LX004** | 1.5 | 1.5 |
| **LX018** | 1.5 | 3.125 |

It was noticed that myxopyronin-A alone did not inhibit the growth of *M. bovis* BCG in the presence of 50% FBS with concentration 4 times higher than its MIC in 100 % medium with no FBS. Surprisingly, on the other hand, the MICs of the LX004, LX018 and ethambutol were not affected by the addition of the FBS. These two combinations (Lx004 and Lx018) retained FIC below 1 even in the presence of FBS.

Among the three RNAPls, myxopyronin, ripostatin and corallopyronin, we found that myxopyronin A combination with Ethambutol is a highly attractive candidate as a new TB drug.

### Inhibition of M. tuberculosis strains with Myxopyronin A- Ethambutol cocktails

We also tested Myxopyronin-A and Ethambutol combinations against virulent drug sensitive and drug resistant *M.tuberculosis* strains. The results showed that Myxopyronin-A Ethambutol cocktails such as Lx004, Lx018, Lx019 efficiently inhibit drug sensitive strains. Most surprisingly, the cocktails also inhibited the growth of most of the Multiple drug resistant(MDR) strains and even some of the Extreme Drug Resistant (XDR) strains. New drugs against MDR and XDR strains are badly needed since current drugs are unable to treat such patients successfully.

**Table 7.**

| Growth of *Mycobacterium tuberculosis* in the presence of Lionex (LX) compounds | | | | | | | |
|---|---|---|---|---|---|---|---|
| Mycobacteriumtuberculosisstrains | Profile | LX018 | LX019 | LX021 | LX024 | Growth Control | Negative Control |
| 09-3194 | S | - | - | - | - | + | - |
| 09-537 | S | - | + | +/- | +_{/}- | + | - |
| 09-3130 | S | - | - | - | - | + | - |
| 09-3193 | S | - | - | - | - | + | - |
| 09-3134 | S | - | - | - | + | + | - |
| 09-3155 | S | - | - | - | - | + | - |
| 09-0371 | S | - | - | + | + | + | - |
| 09-3131 | S | - | - | +/- | +/- | + | - |
| 09-3163 | S | - | - | - | - | + | - |
| 09-3185 | S | - | - | - | - | + | - |
| 09-3528 | S | - | - | + | + | + | - |
| 09-3549 | MDR | - | - | - | +/- | + | - |
| 09-3847 | MDR | +/- | + | + | + | + | - |
| 09-3539 | MDR | - | - | - | - | + | - |
| 09-3275 | MDR | + | + | + | + | + | - |
| 09-3271 | MDR | - | - | + | + | + | - |
| 09-3573 | MDR | +/- | - | + | + | + | - |
| 09-3242 | MDR | - | + | - | - | + | - |
| 09-3463 | MDR | - | - | - | - | + | - |
| 09-3246 | MDR | - | - | - | - | + | - |
| 09-3833 | MDR | - | +/- | +/- | +/- | + | - |
| 08-1783 | MDR | - | +/- | +/- | +/- | + | - |
| 09-3748 | MDR | - | - | - | - | + | - |
| 09-3065 | MDR | - | - | - | - | + | - |
| 09-3474 | MDR | - | - | - | - | + | - |
| 09-3258 | MDR | + | + | + | + | + | - |
| 09-3265 | MDR | - | - | - | - | + | - |
| 11-0621 | MDR | - | - | - | - | + | - |
| 08-1146 | Pre-XDR | - | - | - | - | + | - |
| 08-1074 | Pre-XDR | +/- | +/- | +/- | +/- | + | - |
| | Pre- | | | | | | |
| 08-1468 | XDR | - | - | - | - | + | - |
| | Pre- | | | | | | |
| 08-1535 | XDR | - | - | - | - | + | - |
| | Pre- | | | | | | |
| 08-1468 | XDR | - | - | - | - | + | - |
| 08-0533 | Pre-XDR | + | + | + | + | + | - |
| | Pre- | | | | | | |
| 08-1077 | XDR | - | - | - | - | + | - |
| | Pre- | | | | | | |
| 08-1198 | XDR | - | +/- | +/- | +/- | + | - |
| | Pre- | | | | | | |
| 09-2066 | XDR | + | + | + | + | + | - |
| | Pre- | | | | | | |
| 08-1233 | XDR | + | - | + | + | + | - |
| | Pre- | | | | | | |
| 08-1120 | XDR | - | - | +/- | +/- | + | - |
| | Pre- | | | | | | |
| 08-1174 | XDR | - | - | - | - | + | - |
| 09-3261 | Pre-XDR | - | +/- | +/- | +/- | + | - |
| 07-2328 | XDR | - | - | - | - | + | - |
| 07-1642 | XDR | - | - | NA | - | + | - |
| 07-3503 | XDR | - | - | - | - | + | - |
| 08-1638 | XDR | + | + | + | + | + | - |
| 08-1529 | XDR | - | - | - | - | + | - |
| 08-0589 | XDR | - | - | - | - | + | - |
| 07-3082 | XDR | - | +/- | +/- | + | + | - |
| 07-3216 | XDR | - | + | + | + | + | - |
| 08-1145 | XDR | + | + | + | + | + | - |

| | | | | | | | |
|---|---|---|---|---|---|---|---|
| Legend: + indicatesgrowth - indicatesnogrowth +/- indicates minimal growth S= drug sensitive MDR= multiple drugresistant XDR= Extreme drugresitant | | | | | | | |

### Testing of efficiency of novel drug cocktails in animal experiments

A series of experiments were also performed for testing the efficacy of drug cocktails in mice infected with ***M. tuberculosis*** strain H37Rv. The results confirmed the results mentioned above with the drug cocktails.

### Discussion:

Bacterial DNA-dependent RNA polymerase (RNAP) is an attractive drug target. The inactivation of this crucial enzyme would lead to cell death. In addition, bacterial RNAP does not share extensive sequence homology with eukaryotic RNAP (*Bai et al.*, *2011*). Generally, RNAP assay as a target based screening tool has the advantage of speeding the role of medicinal chemistry to modify the compounds in a way that continue to hit the desired target (*Nuermberger et al.*, 2010). The RNAP assay that was developed by (*Bhuju,* 2009) has many advantages over the previously used RNAP assays. In this study, we used this assay with few modifications. Increasing the concentration of adenosine 5' phosphosulfate (APS) and the time of the reaction catalyzed by the ATP sulfurylase enzyme and using the half area plates improved the reproducibility of the assay. Furthermore, the assay was used to predict the inhibitory effect of different combinations that contain RNAP inhibitors (RNAPIs). The results showed the inhibitory effect of different combinations on the RNAP in a dose related manner.

The absence of cross resistance between rifampicin(RIF) and the RNAPIs myxopyronin, corallopyronin and ripostatin make them very attractive molecules for the researchers in the field of new anti-TB drug discovery. We evaluated the *in vitro* antimycobacterial activity of the three compounds. The results revealed MIC of myxopyronin and corallopyronin 6.25 and 12.5 µg/ml respectively, while ripostatin did not inhibit the bacterial growth up to concentration of 25 µg/ml. with the evaluation of the *in vitro* toxicity profile, corallopyronin showed low selectivity with selectivity index (SI) of 2. In addition, corallopyronin seems to be upon long storage transforms into highly toxic compound which affects the mammalian cell division through its toxic effect on the cellular microtubules leading to large multinucleated cells. We concluded that corallopyronin is not a promising antimycobacterial drug candidate.

On the other hand, myxopyronin A showed higher safety margin with SI greater than 16.

We used our novel strategy of developing drug cocktails using Ethambutol and a new drug such as Myxopyronin A. Indeed, Myxopyronin A showed synergistic effect with the commercially available antimycobacterial agent, ethambutol with FIC of 0.64. In our results, corallopyronin and ripostatin did not show synergism with ethambutol.

Interestingly, the all combinations between Myxopyronin A and Ethambutol showed high antibacterial activity against *M. bovis* BCG with wide safety margin with no detectable effect on the cellular structures with immunocytochemistry (ICC) staining even with concentrations much higher than their MIC. Similar results were obtained with drug sensitive and most of the MDR/XDR strains of *M. tuberculosis.*

Furthermore, by testing the *in vitro* MIC with the presence of 50 % fetal bovine serum (FBS), we found that Myxopyronin A lost its anti-TB activity even with concentrations 4 times higher than its MIC in 100 % medium indicating high binding to proteins. At the same time the combination between Myxopyronin A and Ethambutol retained its low MIC with the presence of 50 % FBS. Hence, we conclude that a proper drug combination containing Myxopyronin A and Ethambutol is highly promising agent for killing tuberculosis causing pathogen. Ethambutol is a cell wall inhibitor and its *in vitro* MIC was not affected by the addition of the FBS. This is matching with the results of *Lee et al.,* (*1977*) who showed that the extent of ethambutol binding to plasma proteins ranges between 20 and 30 %. Ethambutol. Through its action on the cell wall of the microbe may facilitate the entrance of the MyxopyroninA to inhibit the RNAP even with low concentration of the free fraction.

Myxopyronin was discovered more than 30 years ago. It was considered very attractive RNAPI due to the absence of cross-resistance between RIF and myxopyronin. The physiochemical properties of myxopyronin and its high binding to plasma proteins were restrictive factors to enter the subsequent stages of the discovery pathway. The extent of binding to plasma proteins can affect the antimicrobial activity of an antibiotic through different aspects. The bound antibiotic is in a temporary inactive state that cannot hit the microbe. Additionally, high binding to plasma proteins usually limits the distribution of the antibiotic. The percentage of binding determines the rate by which the antibiotic diffuses to the extravascular compartment. And for highly bound drugs, the half life (t_{1/2}) will determine if the drug can reach a satisfactory level in tissues or not (*Rolinson*, *1980).* We show that studying the pharmacokinetic (PK) profiles of myxopyroninin *vivo* and *in vitro,* evaluating the PK/PD parameters of myxopyronin ther with ethambutol in different combinations, and the extent of their binding to proteins as a useful and novel strategy for optimal drug development.

### References:

Aristoff, P. A., Garcia, G. A., Kirchhoff, P. D., Hollis Showalter, H. D. (2010). Rifamycins - obstacles and opportunities. Tuberculosis (Edinburgh, Scotland), 90(2), 94-118.
Artsimovitch, I., Vassylyev, D. G. (2006). Is it easy to stop RNA polymerase?. Cell Cycle, 5(4), 399-404.
Bai, H., Zhou, Y., Hou, Z., Xue, X., Meng, J., Luo, X. (2011) Targeting bacterial RNA polymerase: promises for future antisense antibiotics development. Infectious Disorders Drug Targets, 11(2), 175-87.
Bhat, J., Rane, R., Solapure, S. M., Sarkar, D., Sharma, U., Harish, M. N., Lamb, S., et al. (2006). High-throughput screening of RNA polymerase inhibitors using a fluorescent UTP Analog. Journal of Biomolecular Screening, 11(8), 968-976.
Bhuju, S. (2009). Development of novel drug screening assays and molecular characterization of rifampicin and pyrazinamide resistance in Mycobacterium tuberculosis.Braunschweig University, Ph.D.
Bujnowski, K., Synoradzki, L., Dinjus, E Zevaco, T., Augustynowicz-Kopec, E., Zwolska, Z. (2003). Rifamycin antibiotics F new compounds and synthetic methods. Part 1: Study of the reaction of 3-formylrifamycin SV with primary alkylamines or ammonia. Tetrahedron, 59(11), 1885-1893.
Chao, M. C., Rubin, E. J. (2010). Letting sleeping dos lie: does dormancy play a role in tuberculosis?. Annual Review of Microbiology, 64, 293-311.
Chopra, I. (2007). Bacterial RNA polymerase: a promising target for the discovery of new antimicrobial agents. CurrOpinInvestig Drugs, 8(8), 600-607.
Daniel,V., Grimberg, J., Zeevi, M. (1975). In vito synthesis of tRNA precursors and their conversion to matur size tRNA. Nature, 257, 193-197.
Haebich, D., Von Nussbaum, F. (2009). Lost in transcription-inhibition of RNA polymerase.AngewandteChemie (International ed. in English), 48(19), 3397-3400.
Heep, M., Rieger, U., Beck, D., , Lehn, N. (2000). Mutations in the beginning of the rpoB gene can induce resistance to rifamycins in both Helicobacter pylori and Mycobacterium tuberculosis. Antimicrobial Agents and Chemotherapy, 44(4), 1075-1077.
McClure, W. R. (1980). Rate-limiting steps in RNA chain initiation. Proceedings of the National Academy of Sciences of the United States of America, 77(10), 5634-5638.
Mukhopadhyay, J., Das, K., Ismail, S., Koppstein, D., Jang, M., Hudson, B., Sarafianos, S., et al. (2008). The RNA polymerase " switch region " is a target for inhibitors. Cell, 135(2), 295-307.
Musser, J. M. (1995). Antimicrobial agent resistance in mycobacteria: molecular genetic insights. Clinical Microbiology Reviews, 8(4), 496-514.
Moghazeh, S. L., Pan, X., Arain, T., Stover, C. K., Musser, J. M., , Kreiswirth, B. N. (1996). Comparative antimycobacterial activities of rifampin, rifapentine, and KRM-1648 against a collection of rifampin-resistant Mycobacterium tuberculosis isolates with known rpoB mutations. Antimicrobial Agents and Chemotherapy, 40(11), 2655-2657.
Nuermberger, E. L., Spigelman, M. K., Yew, W. W. (2010). Current development and future prospects in chemotherapy of tuberculosis.Respirology, 15(5), 764-778.
O'Neill, A. O., Oliva, B., Storey, C., Hoyle, A., Fishwick, C., Chopra, I., Chopra, I. A. N. (2000). RNA polymerase inhibitors with activity against rifampin-resistant mutants of staphylococcus aureus RNA polymerase inhibitors with activity against rifampin-resistant mutants of staphylococcus aureus. Antimicrobial Agents And Chemotherapy, 44(11), 3163-3166.
Palomino, J., Martin, A., Camacho, M., Guerra, H., Swings, J., Portaels, F. (2002).Resazurinmicrotiter assay plate: simple and inexpensive method for detection of drug resistance in Mycobacterium tuberculosis. Antimicrobial Agents and Chemotherapy.46(8), 2720-2722.
Rolinson, G. N. (1980). The significance of protein binding of antibiotics in antibacterial chemotherapy. Journal of Antimicrobial Chemotherapy, 6, 311-317.
Römmele, G., Wirz, G., Solf, R., Vosbeck, K., Gruner, J., Wehrli, W. (1990). Resistance of Escherichia Coli to rifampicin and sorangicin A-a comparison. The Journal of Antibiotics (Tokyo), 43 (1), 88-91.
Valim, A. R. M., Rossetti, M. L. R., Marta, O., Zaha, A., Valim, I. A. R. M. (2000). Mutations in the rpoB Gene of Multidrug-Resistant Mycobacterium tuberculosis Isolates from Brazil. Journal of Clinical Microbiology, 38(8), 3119-3122.
Vassiliou, W., Epp, J. B., Wang, B. B., Del Vecchio, A. M., Widlanski, T., Kao, C. C. (2000). Exploiting polymerase promiscuity: A simple colorimetric RNA polymerase assay. Virology, 274(2), 429-437.
Xu, M., Zhou, Y. N., Goldstein, B. P., & Jin, D. J. (2005). Cross-resistance of Escherichia Coli RNA polymerases conferring rifampin resistance to different antibiotics. Journal Of Bacteriology, 187(8), 2783-2792.
Wu, P., Daniel-Issakani, S., LaMarco, K., Strulovici, B. (1997). An automated high throughput filtration assay: Application to polymerase inhibitor identification. Analytical Biochemitry, 245(2), 226-230.
Yuen, L. K. W., Leslie, D., Coloe, P. J. (1999). Bacteriological and Molecular Analysis of Rifampin-Resistant Mycobacterium tuberculosis Strains Isolated in Australia. Journal of Clinical Microbiology, 37(12), 3844-3850.

## Claims

1. Pharmaceutical composition comprising a combination of ethambutol with at least one additional anti-bacterial agent, in particular, anti-mycobacterial agent for use in the treatment of bacterial infection, in particular, mycobacterial infection.

2. The pharmaceutical composition according to claim 1 for use in the treatment of bacterial infection, in particular, mycobacterial infection with a multi drug resistant strain.

3. The pharmaceutical composition according to claim 1 or 2 for use in the treatment of bacterial infection, in particular, mycobacterial infection of an individual infected with a bacterial strain, in particular, mycobacterial strain, being resistant against rifampicin treatment.

4. The pharmaceutical composition according to any one of claims 1 to 3 for use in the treatment of infection with ethambutol sensitive bacterial, in particular, mycobacterial, strains.

5. The pharmaceutical composition according to any one of the preceding claims, wherein the at least one additional anti-bacterial agent, in particular, anti mycobacterial agent is a 2-pyrone containing antibiotic.

6. The pharmaceutical composition according to claim 5 for use in the treatment of bacterial infection, in particular, mycobacterial infection wherein the at least one additional anti-bacterial agent is a compound according to formula I 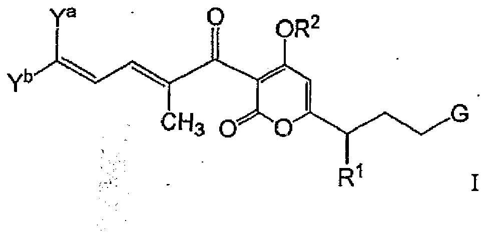 or a pharmaceutically acceptable salt thereof, wherein:
R1 is hydrogen or -CH3;
R2 is hydrogen or -C1-C6 straight or branched alkyl;
one of Ya and Yb is hydrogen or C1-C4 straight alkyl, and the other of Ya and Yb is C1-C10 straight or branched alkyl, C2-C12 straight or branched hydroxyalkyl,
C2-C12 straight or branched alkenyl, C2-C12 straight or branched hydroxyalkenyl, phenyl, C7-C12 (aryl)hydroxyalkyl, C6-C12 heteroalkyl, C6-C12 (heteroaryl)hydroxyalkyl, or Ya and Yb are taken together with their intervening atom to form a 4-6 membered ring having 0-1 ring heteroatoms selected from nitrogen, oxygen or sulphur, said ring optionally substituted by one or two C1-C6 alkyl, C2-C6 alkenyl or hydroxyalkyl groups, wherein an alkyl, aryl or heteroaryl moiety of Ya and Yb optionally is substituted with 1-3 groups independently selected from halo, -C1-C6 hydroxyalkyl, -C1-C4 alkoxy, -C1-C4 trifluororalkoxy, - CN, -C1-C4 alkoxycarbonyl, -C1-C4 alkylcarbonyl, -S(C1-C4 alkyl), and -SO2(C1-C4 alkyl);
G is -CH=CH-NHC(O)-R3, -CH=CH-NHC(S)-R3, -CH2CH2NHC(O)-R3, -CH2CH2NHC(S)-R3, -CH2NHNHC(O)-R3, or-CH2NHNHC(S)-R3;
each R3 is independently C1-C6 alkyl, -O(C1-C6 alkyl), -S(C1-C6 alkyl), or - N(R4)2;
and
each R4 is independently hydrogen or-C1-C6 alkyl;

7. The pharmceutical composition according to any one of claims 1 to 6 wherein the at least one additional anti-bacterial, in particular, anti-mycobacterial agent is an agent selected from ripustatine.

8. The pharmaceutical composition according to any one of the preceding claims for use in the treatment of mycobacterial infection wherein the composition comprises a combination of myxopyronine A or B with ethambutol.

9. A method of improving anti-bacterial activity, in particular, bactericidal or bacteriostatic activity of a drug by combining said drug with an effective concentration of ethambutol.

10. The method according to claim 9 for improving mycobacterial activity against mycobacteria of an anti-mycobacterial drug by combining it with an effective concentration of ethambutol.

11. A method of treating an individual afflicted with a bacterial infection, in particular, a mycobacterial infection including the step of administering a pharmaceutical composition according to any one of claims 1 to 8 to said individual.

12. The method according to claim 11 or the pharmaceutical composition for use in the treatment of bacterial infection, in particular, mycobacterial infection according to any one of claims 1 to 8 wherein the active agents are adapted to be administered simultaneously, separately or sequentially.
